# EUROPÄISCHE PATENTSCHRIFT

(11) **EP 0 760 690 B1**
(45) Veröffentlichungstag und Bekanntmachung des Hinweises auf die Patenterteilung: **23.01.2002**
(21) Anmeldenummer: 96906697.6
(22) Anmeldetag: 22.03.1996
(51) Int. Cl.: A61M 5/142

(54) **IMPLANTIERBARE INFUSIONSPUMPE**
IMPLANTABLE INFUSION PUMP
POMPE A PERFUSION IMPLANTABLE

(30) Priorität: 23.03.1995 DE 19510583
(43) Veröffentlichungstag der Anmeldung: 12.03.1997
(73) Patentinhaber: Tricumed Medizintechnik GmbH, 24143 Kiel (DE)
(72) Erfinder: OTTO, Karl-Heinz, D-24146 Kiel (DE)
(74) Vertreter: Heselberger, Johannes
(86) Internationale Anmeldenummer: DE9600496
(87) Internationale Veröffentlichungsnummer: WO9629105

(56) Entgegenhaltungen:
- EP-A- 0 075 762
- US-A- 4 820 273

## Beschreibung

Die Erfindung betrifft eine implantierbare Infusionspumpe mit den Merkmalen des Oberbegriffs des Anspruchs 1, wie sie beispielsweise aus der DE 26 04 113 C2 bekannt ist. Aus der US 4 820 273 ist weiter eine Infusionspumpe bekannt, bei der eine als "bladder" (Blase) bezeichnete Membran ein Medikamentenraum zu einem Druckraum hin begrenzt.

Die gattungsgemäßen Infusionspumpen werden Patienten implantiert, die einen ständigen Bedarf an Zufuhr eines Arzneimittels haben, beispielsweise Schmerzpatienten und Spastikern. Bisher ist eine Flußregelung immer nur an einer Ausführeinrichtung des Medikaments vorgesehen worden, und es ist stets nur ein einziger Medikamentenbeutel vorgesehen.

Der Erfindung liegt die Aufgabe zugrunde, eine kostengünstig herzustellende Infusionspumpe mit mehreren verschiedenen möglichen Flußraten zu schaffen.

Erfindungsgemäß wird diese Aufgabe durch die Merkmale des Anspruchs 1 gelöst, die Unteransprüche geben vorteilhafte Ausgestaltungen der Erfindung an.

Die Erfindung wird im folgenden anhand einer Zeichnung erläutert. Dabei zeigt:

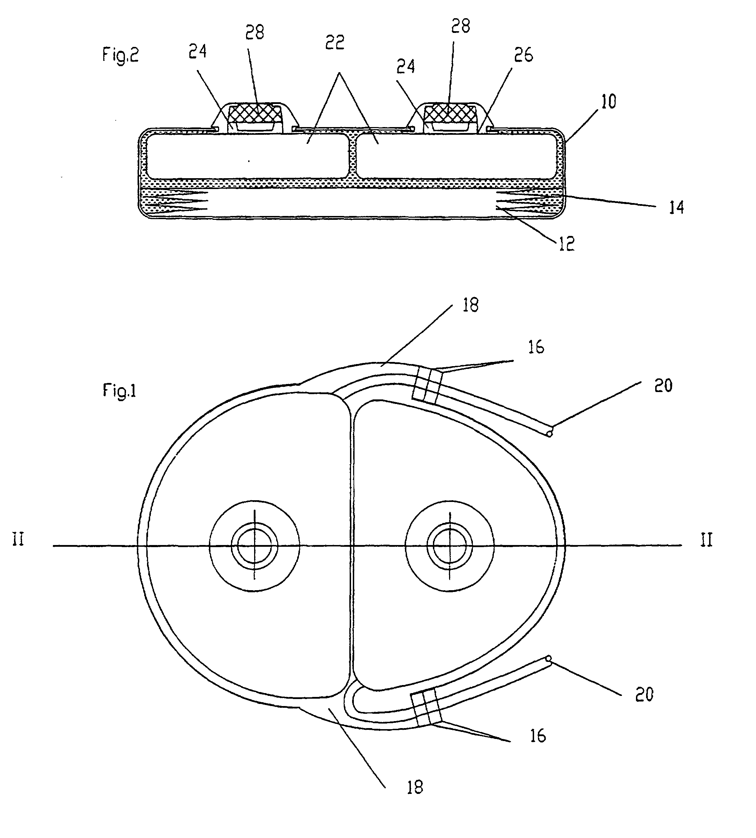

- Fig. 1: eine Draufsicht auf eine solche Infusionspumpe, und
- Fig. 2: eine Schnittansicht entlang der Linie II-II von Fig. 1.

Die Infusionspumpe weist ein Gehäuse 10 auf, das aus Kunststoff gefertigt ist. Das Gehäuse 10 nimmt einen. Balg 12 auf, der ein einen Dampfdruck erzeugendes Treibmittel aufnimmt. Der verbleibende Raum 14 nimmt bei dem gezeigten Ausführungsbeispiel zwei aus einem nachgiebigen Kunststoff gefertigte Beutel 22 auf, die jeweils ein Arzneimittel, beinhalten. Der von den Beuteln 22 nicht belegte Teil des Raumes 14 ist mit einer das Material der Beutel und des Gehäuses nicht angreifenden Flüssigkeit, beispielsweise Glyzerin, gefüllt. Die Flüssigkeit ist dabei so ausgewählt, das sie etwa aus dem Balg 14 austretendes Treibmittel chemisch bindet, damit dieses nicht durch das Kunststoffgehäuse in den Körper des Patienten eindringen kann.

Das Gehäuse 10 trägt weiter eine Drosselstrecke 16, an die sich ein Anschluß für einen Katheter 20 anschließt.

Die nach der Implantation der Infusionspumpe zu der Haut des Patienten weisenden oberen Fläche des Gehäuses 10 ist mit einer der Anzahl der aufzunehmenden Beutel 22 entsprechenden Anzahl von Ausnehmungen 26 versehen, die einen aus einem von einer Nadel durchdringbaren, in das Material der Kunststoff-Beutel 22 eingebrachte erste Stopfen 28 aufnehmen. Durch diese erste Stopfen 28 hindurch können die Kunststoff-Beutel 22 mit dem dem Patienten applizierenden Arzneimittel aufgefüllt werden.

Die Kunststoff-Beutel sind weiter mit in deren Material eingebrachten zweiten Stopfen 24 versehen, die als Kupplungsstück zu der Drosselstrecke 16 hin dienen.

Die von dem Balg 12 aufgenommene einen Dampfdruck erzeugende Flüssigkeit übt einen Druck auf die Beutel 22 aus, wobei dieser Druck durch die diesen umgebende Flüssigkeit gleichmäßig verteilt wird. Bei Eröffnung der Drosselstrecke tritt somit eine definierte Menge des Arzneimittels aus den Beuteln 22 in das Katheter aus und damit in den Körper des Patienten ein.

Bei der hier vorgeschlagenen Ausbildung einer Infusionspumpe sind die Anforderungen an die Reinheit und Sterilität des Innenraums des Gehäuses weitaus geringer, da das zu applizierende Arzneimittel nicht mit der Gehäusewandung selbst in Verbindung tritt. Bei dem bevporzugten Ausführungsbeispiel besteht weiter nicht das Erfordernis der absoluten Dichtigkeit des Gehäuses, da das Arzneimittel ja von dem Beutel aufgenommen wird und das Treibmittel bei Auftreten einer Undichtigkeit des Balgs von der den Beutel umgebenden Flüssigkeit gebunden wird.

## Patentansprüche

1. Implantierbare Infusionspumpe, mit einem Gehäuse (10), das einen eine einen Dampfdruck erzeugendes Treibmittel aufnehmenden Balg (12) aufnimmt und einen ein Arzneimittel aufnehmenden Raum (14) ausbildet und mit einer Drosselstrecke (16) und einem Anschluß (18) für ein Katheter (20) versehen ist,
**dadurch gekennzeichnet, daß**
der Raum (14) wenigstens zwei das Arzneimittel beinhaltende, aus einem nachgiebigen Kunststoff gefertigte Beutel (22) aufnimmt, die jeweils mit einem mit der Drosselstrecke (16) verbundenen Kupplungsstück (24) versehen sind.

2. Infusionspumpe nach Anspruch 1, **dadurch gekennzeichnet, daß** das Gehäuse (10) aus Kunststoff gefertigt ist.

3. Infusionspumpe nach einem der vorangehenden Ansprüche, **dadurch gekennzeichnet, daß** die nach der Implantation zu der Haut des Patienten weisende obere Fläche des Gehäuses (10) mit einer der Anzahl der Beutel (22) entsprechenden Anzahl von Ausnehmungen (16) versehen ist, die einen aus einem von einer Nadel durchdringbaren ersten Stopfen (18) aufnehmen.

4. Infusionspumpe nach einem der vorangehenden Ansprüche, **dadurch gekennzeichnet, daß** das Kupplungsstück (24) als in das Material der Kunststoff-Beutel (22) eingebrachter zweiter Stopfen ausgebildet ist.

5. Infusionspumpe nach einem der vorangehenden Ansprüche, **dadurch gekennzeichnet, daß** der von den Beuteln (22) nicht belegte Teil des Raums (14) mit einer Flüssigkeit gefüllt ist.

6. Infusionspumpe nach einem der vorangehenden Ansprüche, **dadurch gekennzeichnet, daß** die Flüssigkeit die Eigenschaft der Bindung des Treibmittels hat.

## Claims

1. Implantable infusion pump with a housing (10), said housing (10) receiving a bellow (12), said bellow (12) receiving a propellant creating a vapour pressure, and said housing (10) forming a space (14), said space (14) receiving a pharmaceutical, and said housing (10) comprising a throttle track (16) and a connecting means (18) for a catheter (20),
**characterized in that**
said space (14) receives at least two bags (22) made of a flexible synthetic material, said bags (22) containing the pharmaceutical and each comprising a coupling (24) being connected to said throttle track (16).

2. Infusion pump according to claim 1, **characterized in that** said housing (10) is made of synthetic material.

3. Infusion pump according to any of the preceding claims, **characterized in that** the upper surface of said housing (10), facing the skin of the patient after implantation, comprises a number of throttle tracks and recesses (16/26), said number corresponding to the number of bags (22), said throttle tracks and recesses (16/26) receiving a first plug (28) being penetrable by a needle.

4. Infusion pump according to any of the preceding claims, **characterized in that** said coupling (24) is formed as a second plug inserted into the material of said synthetic bags (22).

5. Infusion pump according to any of the preceding claims, **characterized in that** the part of the space (14) which is not occupied by the bags (22) is filled with liquid.

6. Infusion pump according to any of the preceding claims, **characterized in that** said liquid has the characteristic of binding the propellant.

## Revendications

1. Pompe implantable de perfusion, avec un boîtier (10), qui enferme un soufflet (12) enfermant un agent d'entraînement produisant une pression gazeuse, et qui définit un volume (14) enfermant un produit pharmaceutique et qui est pourvu d'une zone d'étranglement (16) et d'un raccord (18) pour un cathéter (20),
**caractérisé en ce que**
le volume (14) inclut au moins deux poches (22) contenant le produit pharmaceutique, réalisées en un plastique souple, qui sont respectivement pourvues d'une pièce de couplage (24) reliée à la zone d'étranglement (16).

2. Pompe de perfusion selon la revendication 1, **caractérisée en ce que** le boîtier (10) est réalisé en plastique.

3. Pompe de perfusion selon l'une des revendications précédentes, **caractérisée en ce que** la surface supérieure du boîtier (10) tournée vers la peau du patient après implantation est pourvue d'un nombre de zones d'étranglements et d'alvéoles (16, 26) correspondant au nombre de poches (22) et qui comprennent un premier obturateur (28) pénétrable par une aiguille.

4. Pompe de perfusion selon l'une des revendications précédentes, **caractérisée en ce que** la pièce de couplage (24) est conformée en un second obturateur incorporé au matériau de la poche en plastique (22).

5. Pompe de perfusion selon l'une des revendications précédentes, **caractérisée en ce que** la partie du volume (14) non recouverte par les poches (22) est remplie d'un liquide.

6. Pompe de perfusion selon l'une des revendications précédentes, **caractérisée en ce que** le liquide a la propriété de se lier à l'agent d'entraînement.
